# FASCICULE DE BREVET EUROPEEN

(11) **EP 2 844 077 B1**
(45) Date de publication et mention de la délivrance du brevet: **10.01.2024**
(21) Numéro de dépôt: 13722343.4
(22) Date de dépôt: 02.05.2013
(51) Int. Cl.: C07C 229/08, A23K 20/10, A23K 20/142, A23K 50/00, A23K 50/10, A23K 50/80

(54) **COMPLEXE ORGANOMÉTALLIQUE, POUDRE DESTINÉE À L'ALIMENTATION ANIMALE ET PROCÉDÉS DE PRÉPARATION**
METALLORGANISCHER KOMPLEX, PULVER FÜR TIERFUTTER UND HERSTELLUNGSVERFAHREN DAFÜR
ORGANOMETALLIC COMPLEX, POWDER INTENDED FOR ANIMAL FEED AND PREPARATION METHODS THEREOF

(30) Priorité: 02.05.2012 FR 1253991
(43) Date de publication de la demande: 11.03.2015
(73) Titulaire: Pancosma S.A., 1218 Le Grand-Saconnex (CH)
(72) Inventeur: RAULET, Christelle, F-74160 Saint julien en genevois (FR); OGUEY, Clémentine, CH-1434 Ependes (CH); BRAVO, David, CH-1400 Yverdon-les Bains (CH)
(74) Mandataire: Moinas & Savoye SARL
(86) Numéro de dépôt international: PCT/EP2013/059173
(87) Numéro de publication internationale: WO 2013/164416

(56) Documents cités:
- WO-A1-83/01559
- WO-A2-03/072048
- FR-A1- 2 833 187
- US-A- 5 185 144
- US-A1- 2004 137 108
- GINGASU D ET AL: "Copper ferrite obtained by two ''soft chemistry'' routes", JOURNAL OF ALLOYS AND COMPOUNDS, ELSEVIER SEQUOIA, LAUSANNE, CH, vol. 425, no. 1-2, 30 novembre 2006 (2006-11-30), pages 357-361, XP028000256, ISSN: 0925-8388, DOI: 10.1016/J.JALLCOM.2006.01.026 [extrait le 2006-11-30]
- MCARDLE N T ET AL: "Ultrastructural studies of the effects produced by some amino acid metal systems on escherichia coli B", INORGANICA CHIMICA ACTA, ELSEVIER BV, NL, vol. 92, no. 2, 1984, pages 113-121, XP026595770, ISSN: 0020-1693, DOI: 10.1016/S0020-1693(00)80007-9 [extrait le 1984-06-01]
- S. BUNEL ET AL: "Origin of chirarity in charge transfer bands of Cu(II) acidates", JOURNAL OF INORGANIC AND NUCLEAR CHEMISTRY, vol. 43, 1981, pages 971-975, XP002691983, Pergamon Press, GB ISSN: 0022-1902
- ATSUSHI TOYOTA ET AL: "A Rock-Salt-Like Lattice Structure Consisting of Monocationic and Monoanionic AuIAgICuII Supramolecular Cages ofD-Penicillaminate", ANGEWANDTE CHEMIE INTERNATIONAL EDITION, vol. 44, no. 7, 4 February 2005 (2005-02-04), pages 1088-1092, XP055430254, ISSN: 1433-7851, DOI: 10.1002/anie.200462205
- SHARADA P. KAIWAR ET AL: "Synthesis, characterization and DNA interaction studies of CrIII products isolated from CrVI reduction with -SH containing molecules", POLYHEDRON., vol. 15, no. 5-6, 1 March 1996 (1996-03-01), pages 765-774, XP055430309, GB ISSN: 0277-5387, DOI: 10.1016/0277-5387(95)00359-7

## Description

L'invention concerne un complexe organométallique du type acide aminé/métal. Ce complexe est pluri-métallique, il peut être cristallin et peut être obtenu sous la forme d'une poudre homogène permettant d'optimiser l'alimentation animale. L'invention se rapporte également à des procédés de préparation d'une telle poudre.

### Arrière-plan de l'invention

Actuellement, pour supplémenter l'alimentation des animaux en métaux tels que le fer, le cuivre, le zinc ou le manganèse, on y ajoute certains composés. Ces composés peuvent être fournis sous une forme organique ou complexée, plus biodisponible que les formes inorganiques. Ils sont employés sous forme de poudre ou liquide et mélangés à dose réduite à la nourriture des animaux pour rééquilibrer ou adapter les rations ou les compléments alimentaires en oligo-éléments.

De tels composés font l'objet de la demande de brevet français publiée sous le numéro FR 2 833 187. Ils se présentent sous forme de complexe d'un acide aminé avec un sulfate de métal. L'un des objectifs visés à la page 4, lignes 3 à 6 de ce document est d'obtenir des grains sous forme de cristaux. Selon une application mentionnée page 4, lignes 14 à 16 de ce document, le composé est constitué d'eau et d'un complexe 1:1 d'acides aminés et de l'un des métaux ou d'un sulfate de l'un des métaux suivants : Cu, Co, Ca, Mg, Mo, Fe, Zn, Cr et Mn. Les exemples décrivent la préparation de composés à base de Zn, Cu, Fe ou Mn. La revendication 11 laisse entendre que certains composés à base de Zn sont du type polymère organométallique.

La demande internationale publiée sous le numéro WO 03/049850 décrit la préparation de glycinates de Mg, Co, Fe, Mn, Cu ou Zn.

Chacun des composés décrits dans ces documents apporte un seul métal. Ceci signifie qu'à chaque métal correspond un composé différent et implique qu'il faut procéder à plusieurs ajouts individuels et donc à autant de dosages séparés pour obtenir une ration alimentaire supplémentée et équilibrée en plusieurs métaux. Le document WO83/01559 décrit un complexe glycinate de cuivre et de zinc. Par conséquent, l'homogénéité des composés dans la ration n'est pas nécessairement totalement optimale. De plus, cela suppose qu'il faut disposer de stocks de chacun des composés et d'une logistique en conséquence.

### Exposé sommaire de l'invention

Le but de l'invention est, notamment, de simplifier et d'optimiser l'homogénéité de la supplémentation en oligo-éléments des rations alimentaires animales et de réduire la logistique correspondante.

Selon l'invention, ce but est atteint en remplaçant l'utilisation de plusieurs composés apportant chacun un métal par l'emploi d'un seul composé fournissant à lui seul tous les métaux souhaités, et ce, dans les proportions souhaitées. Ceci est obtenu grâce à un complexe organométallique selon les revendications.

Ainsi, seuls un dosage et un ajout sont désormais nécessaires pour préparer la ration alimentaire animale. De plus, il n'y plus qu'un seul composé à stocker.

Par ailleurs, ce procédé permet d'optimiser les ratios d'oligo-éléments à supplémenter au sein d'une même particule, et donc non seulement d'homogénéiser au mieux les minéraux et leurs ratios mais pourrait aussi permettre de réduire au mieux les éventuels antagonismes entre minéraux.

Selon un autre aspect, l'invention concerne une poudre contenant des particules comprenant chacune au moins un complexe organométallique selon l'invention.

Selon encore un autre aspect, l'invention a trait à un procédé de préparation de la poudre précitée selon les revendications.

La poudre obtenue par l'un ou l'autre des procédés selon l'invention a l'avantage d'être homogène et cristalline.

De plus, elle n'est pas constituée de particules comprenant un premier métal, de particules comprenant un deuxième métal, et éventuellement de particules comprenant un troisième métal, un quatrième, etc., mais de particules dont chacune comporte à elle seule les premier et deuxième métaux et éventuellement le troisième métal, le quatrième, etc.

D'autres caractéristiques et avantages de l'invention vont maintenant être décrits en détail dans l'exposé suivant qui est donné en référence à la figure annexée qui représente schématiquement :

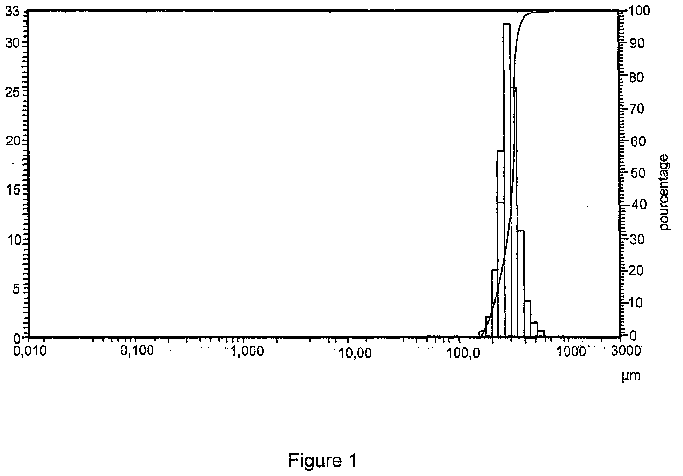

- figure 1 : un diagramme représentant la répartition granulométrique des particules obtenues à l'aide du process décrit dans l'exemple 1.

### Exposé détaillé de l'invention

### Complexe selon l'invention

Le complexe selon l'invention est constitué d'au moins deux acides aminés avec au moins deux métaux différents.

Comme acides aminés, on peut citer la méthionine, la lysine, qui ne font pas partie de l'invention, et la glycine.

Les métaux sont le Cu, Co, Ca, Mg, Mo, Fe, Zn, Cr ou Mn. De préférence, les deux métaux ou plus contenus dans le composé sont choisis dans le groupe constitué par Zn, Cu, Fe et Mn.

Ces métaux peuvent être initialement liés à un ligand présent ou non dans le complexe. Il s'agit généralement d'un sulfate, qui peut être lié à un métal présent sous plusieurs degrés d'oxydation possibles (Cu I ou II, Fe II ou III, ...)

Les composés qui se sont révélés particulièrement intéressants sont ceux à base de glycine, de sulfate de zinc, de sulfate de cuivre, de sulfate de fer, de sulfate de manganèse.

Selon l'invention, les rapports entre les différentes teneurs massiques des métaux peuvent être très variés.

A titre d'exemple, on peut mentionner un composé ayant les rapports massiques suivants :
- masse de Cu/masse de Fe : 0,1111
- masse de Zn/masse de Fe : 0,8888
- masse de Mn/masse de Fe : 0,5555

Un des avantages procurés par l'invention est qu'il est possible de définir, lors de la préparation du complexe, des proportions particulières des métaux que l'on souhaite qu'il contienne. Ces proportions particulières peuvent être celles requises pour couvrir les apports journaliers nécessaires d'un animal donné, ou bien ils peuvent être fixés suivant un cahier des charges fourni par un utilisateur ou pour une utilisation spéciale.

De plus, le ratio molaire entre le(s) acide(s) aminé(s) et le métal est de 1 pour 1. En effet, dans le complexe selon l'invention, un métal est ainsi lié à deux acides aminés et un élément du groupe acide aminé est lié à deux métaux.

Le résultat est donc une molécule se présentant sous la forme d'une chaine alternant un métal et un acide aminé. Cette molécule intègre les différents métaux choisis, dans leur proportion prédéfinie initialement.

Le fait de choisir un ratio molaire de un pour un entre le métal et l'acide aminé présente les avantages suivants :
- cela permet d'éviter d'obtenir un surplus de métaux ou d'acides aminés dans le complexe final, qui seraient libres et pourraient entraîner une mauvaise répartition du métal dans le produit ;
- cela permet d'obtenir 100% d'éléments actifs au travers de la formation d'un seul composé ;
- cela permet d'obtenir un avantage économique à la formulation, la préparation et le sprayage, et facilite son transport.

### Procédés de préparation de la poudre selon l'invention

### a) Premier procédé de préparation

La poudre selon l'invention peut être préparée selon le premier procédé qui permet son obtention à partir de bases multiples.

Ce procédé a l'avantage de pouvoir être mis en oeuvre à température ambiante.

La préparation des solutions aqueuses mentionnées aux étapes a), b) et éventuellement c) s'effectue de façon connue de l'homme du métier, par exemple, suivant les enseignements de la demande internationale précitée n° WO 03/049850 ou de la demande de brevet européen n° EP 2 843 752. Dans ces solutions aqueuses, on respecte le ratio molaire d'un métal pour un élément du groupe acide aminé.

Le flux gazeux est avantageusement un flux d'air.

La pulvérisation est réalisée de façon connue de l'homme du métier.

### b) Deuxième procédé de préparation

Ce procédé permet de partir d'une base unique.

Le mélange de l'étape h) peut avantageusement être réalisé à température ambiante.

Le séchage peut être réalisé dans une étuve et à l'air libre.

Quel que soit le procédé utilisé, les proportions respectives des métaux sont avantageusement choisies de façon à complémenter la ration alimentaire de sorte que celle-ci couvre les besoins de l'animal.

De plus, quel que soit le procédé de préparation utilisé, le produit obtenu se présente sous la forme d'une poudre homogène constituée de particules résultant d'une complexation d'au moins un acide aminé avec tous les métaux ou leurs dérivés utilisés.

Dans l'invention, on utilise de la glycine, les métaux sont liés organiquement à la glycine. Précisément, la glycine est finalement liée aux métaux par les deux oxygènes de son groupe carbonyle. Deux métaux différents peuvent ainsi être liés à la même glycine. Ceci est potentiellement intéressant car l'absorption de chaque métal par l'animal pourrait s'en trouver améliorée. De plus, ce polymère cristallise sous la forme d'un cristal de polymère. Ceci a l'avantage de permettre d'identifier sa structure et éventuellement d'améliorer sa stabilité.

### Utilisations

La poudre selon l'invention est destinée à l'alimentation animale, notamment des ruminants, des monogastriques et des espèces aquacoles.

Elle peut être utilisée à raison de 1 à 1000 g par tonne de nourriture animale, soit dans le but de réaliser un apport journalier de métaux, par exemple, pour favoriser la croissance de l'animal, soit plus ponctuellement, en vue de pallier une carence en métaux constatée chez l'animal ou dans le cadre d'un régime spécifique.

### Exemples

### Exemple 1

Dans cet exemple, on prépare une poudre selon le premier procédé, c'est-à-dire le procédé à bases multiples.

On prépare, séparément, des solutions aqueuses de glycinate de Mn, Zn et Cu.

Les 3 solutions aqueuses de glycinate sont ensuite réunies dans un seul récipient et mélangées.

Le contenu du récipient est ensuite prélevé pour être pulvérisé dans un flux d'air.

Le réglage de l'appareillage est effectué de manière à obtenir une poudre composée de particules dont la formulation après analyse est la suivante :

| Composé mesuré | Teneur en % massique dans la particule |
|---|---|
| Sulfate de Zn, H₂0 | 40,83 |
| Sulfate de Mn, H₂0 | 23,11 |
| Sulfate de Cu, 5 H₂0 | 6,67 |
| Glycine | 29,39 |
| Total | 100 |

La répartition granulométrique des particules est représentée sur le diagramme de la figure 1. Ses valeurs caractéristiques sont les suivantes :
- 10% des particules ont une taille inférieure à 164,42 µm ;
- la taille médiane des particules est de 247,85 µm ; et
- 90% des particules ont une taille inférieure à 388,29 µm.

Des analyses par diffraction des rayons X de la poudre montrent que le produit contient différentes structures cristallines.

### Exemple 2

Dans cet exemple, on a préparé une poudre selon le deuxième procédé, c'est-à-dire celui à base unique.

On prépare une solution aqueuse dans laquelle on mélange dans un récipient du sulfate de Mn, du sulfate de Fe, du sulfate de Zn et du sulfate de Cu, de la glycine.

La formulation de base est la suivante :

| Composé mesuré | Teneur en % massique |
|---|---|
| Sulfate de Fe, 7 H₂0 | 24,32 |

| | |
|---|---|
| Sulfate de Zn, H₂0 | 15,09 |
| Sulfate de Mn, H₂0 | 14,68 |
| Sulfate de Cu, 5 H₂0 | 20,96 |
| Glycine | 24,95 |
| Total | 100 |

Le contenu du récipient est séché à l'étuve.

On obtient ainsi des cristaux de 0,5 à 3 mm bien visibles à l'œil nu (du type cristaux de sel). Les structures cristallines sont proches de celles des particules de l'exemple 1.

## Revendications

1. Complexe organométallique **caractérisé en ce qu'**il est constitué d'au moins deux acides aminés, et d'au moins deux métaux différents, le ratio molaire acide aminé par métal étant de 1 pour 1, dans lequel l'acide aminé est la glycine, et les métaux sont choisis dans le groupe constitué par le zinc, le cuivre, le fer, le manganèse, le cobalt, le calcium, le molybdène, le chrome et le magnésium.

2. Complexe selon la revendication 1, **caractérisé en ce que** chaque métal du complexe organométallique est lié à deux éléments du groupe acide aminé et **en ce que** chaque élément du groupe acide aminé est lié à deux métaux.

3. Complexe selon la revendication 1 ou 2, **caractérisé en ce qu'**il comprend une glycine liée à deux métaux différents par les deux oxygènes de son groupe carbonyle.

4. Complexe selon l'une des revendications précédentes, dans lequel les métaux peuvent être initialement liés à un ligand présent ou non dans le complexe final.

5. Complexe selon la revendication précédente, dans lequel le ligand est un sulfate.

6. Complexe selon l'une des revendications précédentes, constitué de glycine avec du sulfate de zinc, du sulfate de cuivre, du sulfate de fer et du sulfate de manganèse.

7. Poudre comportant des particules comprenant chacune au moins un complexe organométallique, selon l'une des revendications précédentes.

8. Procédé de préparation d'une poudre selon la revendication 7, comportant les étapes suivantes :
a) on prépare une première solution aqueuse d'au moins un acide aminé et d'au moins un premier métal comprenant un ratio molaire acide aminé par métal de 1 pour 1 ;
b) on prépare une deuxième solution aqueuse d'au moins un acide aminé et d'au moins un deuxième métal comprenant un ratio molaire acide aminé par métal de 1 pour 1 ;
c) on répète éventuellement l'étape a) ou b) avec d'autres métaux afin de préparer d'autres solutions aqueuses ;
d) on mélange ensemble toutes les solutions aqueuses préparées ;
e) on réalise un flux gazeux ;
f) on pulvérise le mélange obtenu à l'étape d) dans le flux gazeux ; et
g) on récupère la poudre obtenue.

9. Procédé de préparation d'une poudre selon la revendication 7, comportant les étapes suivantes :
h) on prépare une solution aqueuse dans laquelle on mélange dans un même récipient au moins un acide aminé, au moins un premier métal et au moins un deuxième métal et éventuellement d'autres métaux, la solution comprenant un ratio molaire acide aminé par métal de 1 pour 1 ;
i) on sèche le mélange obtenu à l'étape h) ; et
j) on récupère la poudre obtenue.

10. Utilisation d'une poudre selon la revendication 7 dans l'alimentation animale.

## Patentansprüche

1. Metallorganischer Komplex, **dadurch gekennzeichnet, dass** er aus mindestens zwei Aminosäuren und mindestens zwei verschiedenen Metallen besteht, wobei das Molverhältnis von Aminosäure zu Metall 1 zu 1 beträgt, wobei es sich bei der Aminosäure um Glycerin handelt und die Metalle aus der Gruppe bestehend aus Zink, Kupfer, Eisen, Mangan, Cobalt, Calcium, Molybdän, Chrom und Magnesium ausgewählt sind.

2. Komplex nach Anspruch 1, **dadurch gekennzeichnet, dass** jedes Metall des metallorganischen Komplexes an zwei Elemente der Aminosäuregruppe gebunden ist und jedes Element der Aminosäuregruppe an zwei Metalle gebunden ist.

3. Komplex nach Anspruch 1 oder 2, **dadurch gekennzeichnet, dass** er ein Glycin umfasst, das über die beiden Sauerstoffatome seiner Carbonylgruppe an zwei verschiedene Metalle gebunden ist.

4. Komplex nach einem der vorhergehenden Ansprüche, wobei die Metalle anfänglich an einen Liganden gebunden sein können, der in dem letztendlichen Komplex vorliegt oder nicht.

5. Komplex nach dem vorhergehenden Anspruch, wobei es sich bei dem Liganden um ein Sulfat handelt.

6. Komplex nach einem der vorhergehenden Ansprüche, bestehend aus Glycin mit Zinksulfat, Kupfersulfat, Eisensulfat und Mangansulfat.

7. Pulver, umfassend Teilchen, die jeweils mindestens einen metallorganischen Komplex nach einem der vorhergehenden Ansprüche umfassen.

8. Verfahren zur Herstellung eines Pulvers nach Anspruch 7, das die folgenden Schritte umfasst:
a) Herstellen einer ersten wässrigen Lösung mindestens einer Aminosäure und mindestens eines ersten Metalls mit einem Molverhältnis von Aminosäure zu Metall von 1 zu 1;
b) Herstellen einer zweiten wässrigen Lösung mindestens einer Aminosäure und mindestens eines zweiten Metalls mit einem Molverhältnis von Aminosäure zu Metall von 1 zu 1;
c) gegebenenfalls Wiederholen von Schritt a) oder b) mit anderen Metallen zur Herstellung anderer wässriger Lösungen;
d) Zusammenmischen aller hergestellten wässrigen Lösungen;
e) Herstellen eines Gasstroms;
f) Einsprühen der in Schritt d) erhaltenen Mischung in den Gasstrom; und
g) Gewinnen des erhaltenen Pulvers.

9. Verfahren zur Herstellung eines Pulvers nach Anspruch 7, das die folgenden Schritte umfasst:
h) Herstellen einer wässrigen Lösung, wobei man in demselben Behälter mindestens eine Aminosäure, mindestens ein erstes Metall und mindestens ein zweites Metall und gegebenenfalls andere Metalle mischt, wobei die Lösung ein Molverhältnis von Aminosäure zu Metall von 1 zu 1 aufweist;
i) Trocknen der in Schritt h) erhaltenen Mischung; und
j) Gewinnen des erhaltenen Pulvers.

10. Verwendung eines Pulvers nach Anspruch 7 bei der Tierernährung.

## Claims

1. An organometallic complex, **characterized in that** it consists of at least two elements of amino acid and at least two different metals, the molar ratio of amino acid per metal being 1-to-1, wherein the amino acid is glycine, and the metals are chosen from the group consisting of zinc, copper, iron, manganese, cobalt, calcium, molybdenum, chromium and magnesium.

2. The complex as claimed in claim 1, **characterized in that** each metal of the organometallic complex is bonded to two elements of the amino acid and **in that** each element of the amino acid group is bonded to two metals.

3. The complex as claimed in claim 1 or 2, **characterized in that** it comprises a glycine bonded to two different metals via the two oxygens of its carbonyl group.

4. The complex as claimed in one of the preceding claims, wherein the metals can be initially bonded to a ligand present or not in the final complex.

5. The complex as claimed in the preceding claim, wherein the ligand is a sulfate.

6. The complex as claimed in one of the preceding claims, consisting of glycine with zinc sulfate, copper sulfate, iron sulfate and manganese sulfate.

7. A powder comprising particles each comprising at least one organometallic complex, as claimed in one of the preceding claims.

8. A process for preparing a powder as claimed in claim 7, comprising the following steps:
a) a first aqueous solution of at least one amino acid and of at least one first metal comprising a molar ratio of amino acid per metal of 1-to-1 is prepared;
b) a second aqueous solution of at least one amino acid and of at least one second metal comprising a molar ratio of amino acid per metal of 1-to-1 is prepared;
c) step a) or b) is optionally repeated with other metals in order to prepare other aqueous solutions;
d) all the aqueous solutions prepared are mixed together;
e) a gaseous stream is produced;
f) the mixture obtained in step d) is sprayed into the gaseous stream; and
g) the powder obtained is recovered.

9. A process for preparing a powder as claimed in claim 7, comprising the following steps:
h) an aqueous solution is prepared in which at least one amino acid, at least one first metal and at least one second metal and, optionally, other metals, the solution comprising a molar ratio of amino acid per metal of 1-to-1, are mixed in the same container;
i) the mixture obtained in step h) is dried; and
j) the powder obtained is recovered.

10. The use of a powder as claimed in claim 7 in the feeding of animal.
